(19) Europäisches Patentamt
European Patent Office
Office européen des brevets

(11) **EP 2 828 796 B1**

(12) **FASCICULE DE BREVET EUROPEEN**

(45) Date de publication et mention
de la délivrance du brevet:
**30.11.2022 Bulletin 2022/48**

(21) Numéro de dépôt: **13711430.2**

(22) Date de dépôt: **22.03.2013**

(51) Classification Internationale des Brevets (IPC):
**G06K 9/00** *(2022.01)* **A61M 16/00** *(2006.01)*

(52) Classification Coopérative des Brevets (CPC):
**A61M 16/0051; A61M 16/021; G06K 9/00496; G06K 9/00536**

(86) Numéro de dépôt international:
**PCT/EP2013/056138**

(87) Numéro de publication internationale:
**WO 2013/139979 (26.09.2013 Gazette 2013/39)**

(54) **PROCÉDÉ DE DÉTECTION D'AU MOINS UNE ANOMALIE DANS UN SIGNAL OBSERVÉ, PRODUIT PROGRAMME D'ORDINATEUR ET DISPOSITIF CORRESPONDANTS**

VERFAHREN ZUR ERFASSUNG MINDESTENS EINER ANOMALIE IN EINEM BEOBACHTETEN SIGNAL, COMPUTERPROGRAMMPRODUKT UND ENTSPRECHENDE VORRICHTUNG

METHOD FOR DETECTING AT LEAST ONE ANOMALY IN AN OBSERVED SIGNAL, COMPUTER PROGRAM PRODUCT AND CORRESPONDING DEVICE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **23.03.2012 FR 1252660**

(43) Date de publication de la demande:
**28.01.2015 Bulletin 2015/05**

(73) Titulaires:
• **Université de Bretagne Occidentale**
**29238 Brest Cedex 3 (FR)**
• **Institut Mines-Telecom**
**29238 Brest (FR)**

(72) Inventeurs:
• **LELLOUCHE, François**
**Quebec, Quebec G3B OE8 (CA)**
• **L'HER, Erwan**
**29200 Brest (FR)**
• **PASTOR, Dominique**
**29280 Plouzane (FR)**
• **NGUYEN, Quang-Thang**
**29238 Brest Cedex 3 (FR)**

(74) Mandataire: **Vidon Brevets & Stratégie**
**16B, rue de Jouanet**
**BP 90333**
**35703 Rennes Cedex 7 (FR)**

(56) Documents cités:
**EP-A1- 2 281 506 US-A1- 2005 232 096**

• **Thibault Lamotte: "detection et caracterisation en temps differe de sauts de dynamique: application à la segmentation de l'EMG", , 1 janvier 1995 (1995-01-01), 1 janvier 1995 (1995-01-01), pages 1189-1192, XP055055952, Extrait de l'Internet: URL:http://documents.irevues.inist.fr/bits tream/handle/2042/12408/AR22_2.pdf?sequenc e=1 [extrait le 2013-03-11]**

**Description**

## 1. DOMAINE DE L'INVENTION

Le domaine de l'invention est celui du traitement d'un signal observé via un capteur de mesures.

Plus précisément, l'invention concerne une technique de détection d'au moins une anomalie présente dans le signal observé et qui est liée à l'occurrence d'un phénomène physique non-prédictible.

L'invention a de nombreuses applications, telles que par exemple dans le domaine de la médecine, où elle peut être mise en œuvre dans des dispositifs de surveillance de l'évolution de paramètres physiologiques d'un patient.

Plus généralement, elle peut s'appliquer dans tous les cas où une détection d'une anomalie d'un signal représentatif de l'évolution de paramètres physiques s'avère être importante vis-à-vis des opérations correctrices à effectuer par la suite.

## 2. ARRIÈRE-PLAN TECHNOLOGIQUE

On s'attache plus particulièrement dans la suite de ce document à décrire la problématique existant dans le domaine de la surveillance du débit respiratoire pour un patient ventilé artificiellement, à laquelle ont été confrontés les inventeurs de la présente demande de brevet. Pour rappel, le débit respiratoire correspond au volume d'air circulant dans les poumons par unité de temps. L'invention ne se limite bien sûr pas à ce domaine particulier d'application, mais présente un intérêt pour toute technique de surveillance devant faire face à une problématique proche ou similaire. En effet, la présente technique peut être utilisée pour détecter des anomalies (encore appelées irrégularités ou déviations) par rapport au comportement « normal» (i.e sans anomalies) d'un quelconque des signaux suivants :

- électrocardiogrammes (ECG) qui sont des signaux représentatifs de l'évolution du potentiel électrique qui commande l'activité musculaire du cœur d'un patient, en fonction du temps, mesurée par des électrodes placées à la surface de la peau du patient ;
- électroencéphalogrammes (EEG) qui sont des signaux représentatifs de l'évolution de l'activité électrique du cerveau, en fonction du temps, mesuré par des électrodes placées sur le cuir chevelu d'un patient ;
- signaux représentatifs de l'évolution de la pression artérielle en fonction du temps ;
- signaux représentatifs de l'évolution de la concentration en oxygène en fonction du temps dans des tissus;
- signaux représentatifs de l'évolution de la pression intracrânienne.

Cette liste n'est, bien entendu, pas exhaustive et ne saurait limiter la présente invention à ces seuls domaines d'application. En effet, elle peut être appliquée à tout signal représentatif de l'évolution de paramètres physiologiques d'un patient.

Dans le domaine de la surveillance médicale et de la respiration artificielle, un paramètre vital pour lequel une surveillance particulière doit être réalisée est la surveillance des courbes de débit et de pression au niveau des voies aériennes. En effet, en cas d'une expiration incomplète ou limitée, en particulier chez des patients insuffisants respiratoires chroniques obstructifs ou asthmatiques, un phénomène de capture d'air engendrant une surdistension thoracique peut survenir. Ainsi, la pression des poumons en fin d'expiration augmente (autoPEEP pour Pression Expiratoire Positive intrinsèque) lorsqu'un tel phénomène se produit. La présence d'une distension thoracique se traduit aussi par une absence de retour à zéro du signal de débit respiratoire avant que ne débute l'inspiration suivante.

Ce phénomène de distension thoracique survient chez environ 40 % des patients sous ventilation artificielle (ou mécanique), et il est susceptible d'avoir de multiples conséquences physiologiques délétères. Dépendant du niveau de résistance et de compliance du système respiratoire du patient, et donc de sa constante de temps, une distension thoracique cliniquement significative peut survenir de façon progressive, dans un délai de l'ordre de quelques minutes.

Pour rappel, l'objectif de la ventilation artificielle (ou ventilation mécanique) est d'assister ou de remplacer la respiration spontanée d'un patient si celle-ci devient inefficace ou, dans certains cas, totalement absente. La ventilation artificielle est pratiquée principalement dans le cas de soins critiques (médecine d'urgence, soins intensifs ou intermédiaires), mais est aussi utilisée pour des soins à domicile chez des patients présentant une insuffisance respiratoire chronique.

Dès lors, la détection d'une distension thoracique (i.e la détection d'un auto-PEEP) s'avère importante pour que le praticien (ou clinicien) puisse entreprendre les actions nécessaires permettant de réduire ce phénomène (par exemple en modifiant des réglages du ventilateur, et en allongeant le temps expiratoire).

L'autoPEEP ne peut être quantifiée que de façon ponctuelle par la réalisation d'une pause expiratoire permettant de mesurer la pression d'équilibre télé-expiratoire.

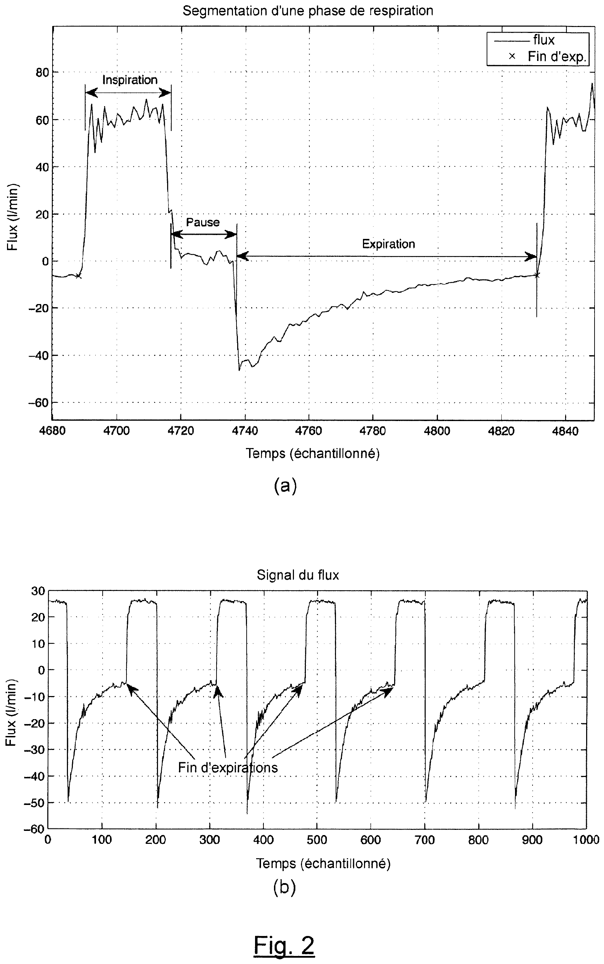

L'évolution de la pression intrapulmonaire peut être cependant déduite de l'analyse du signal représentant l'évolution du débit d'air (en *L/min*) (i.e de l'évolution du volume d'air inspiré et expiré par le patient en fonction du temps, encore appelée courbe de débit respiratoire) d'un patient mesuré via des capteurs positionnés, par exemple, au niveau du ventilateur. Dès lors une distension thoracique (i.e un auto-PEEP) peut être détectée via l'étude d'un tel signal. Les figures 2(a) et 2(b) présentent respectivement les phases caractéristiques (ou segments) d'un tel signal au cours d'un cycle de respiration comprenant un inspiration, une pause et une expiration, et un signal représentatif de l'évolution du débit respiratoire d'un patient en fonction du temps, qui comprend une pluralité de cycles de respirations.

On connaît dans l'état de l'art, une première technique décrite dans le document US2010147305, intitulé « *System and Method for the Automatic Detection of the Expiratory Flow Limitation* » , qui permet d'obtenir, via un traitement automatisé la détection d'une limitation de débit chez le patient.

Cependant cette technique présente différents inconvénients, dont en particulier celui de nécessiter l'intégration de multiples capteurs (espace mort important) ainsi que l'utilisation de variations régulières des paramètres ventilatoires pour permettre cette mesure. Si ce système peut être envisagé en ventilation spontanée et au cours d'une exploration fonctionnelle respiratoire, son utilisation au sein d'un circuit de ventilation artificielle semble plus complexe. Par ailleurs, il ne semble pas à même de permettre une analyse continue et séquentielle de la survenue du phénomène de distension et n'est pas adapté à la détection d'une distension thoracique liée à un problème d'interface entre le patient et le ventilateur.

On connaît aussi dans l'état de l'art une autre technique, appliquée à la détection d'anomalies dans des courbes présentant l'évolution du taux de glucose présent dans le sang d'un patient, décrite dans le document intitulé« Automatic Detection of Anomalies in Blood Glucose Using a Machine Learning Approach » de Y. Zhu, publié dans la revue « IEEE International Conference on Information Reuse and Integration (IRI), 2010 » que l'Homme du métier aurait pu appliquer à notre cas d'espèce.

De plus, on connaît aussi dans l'état de l'art une autre technique, appliquée à la détection d'anomalies dans des électroencéphalogrammes décrite dans le document intitulé« Semi-Supervised Anomaly Detection for EEG Waveforms Using Deep Belief Nets » de Wulsin et al., publié dans les annales de la conférence « Ninth International Conférence on Machine Learning and Applications (ICMLA), 2010 » que l'Homme du métier aurait pu appliquer à notre cas d'espèce.

Enfin, on connaît aussi dans l'état de l'art une autre technique appliquée à la détection d'anomalies décrite dans le document intitulé« Method for automatic detection of wheezing in lung sounds » de R.J. Riella et al., dans la revue « Brazilian Journal of Médical and Biological Research (2009) 42: 674-684 » que l'Homme du métier aurait pu appliquer à notre cas d'espèce.

Un inconvénient majeur de ces techniques réside dans le fait qu'elles nécessitent de mettre en œuvre une phase d'apprentissage à partir d'une première base de données, suivie d'une phase de validation utilisant une deuxième base de données indépendante de la première.

On notera également les techniques de l'état de l'art suivantes:

- Thibault Lamotte: "Détection et caractérisation en temps différé de sauts de dynamique: application à la segmentation de l'EMG", 1 janvier 1995, pages 1189-1192,
- EP 2 281 506 A1 (FICO MIRRORS SA [ES]) 9 février 2011 (2011-02-09).

Une technique de détection d'anomalie est également proposée dans US 2005/232096 A1 (VAN HELVOIRT JAN [NL] ET AL) 20 octobre 2005 (2005-10-20). Cependant, cette détection s'applique à un signal de balayage de disque et non à un signal physique représentatif d'une évolution de paramètres physiologiques d'un patient.

## 3. OBJECTIFS DE L'INVENTION

L'invention, dans au moins un mode de réalisation, a notamment pour objectif de pallier ces différents inconvénients de l'état de la technique.

Plus précisément, dans au moins un mode de réalisation de l'invention, un objectif est de fournir une technique de détection d'anomalie dans un signal (courbe de débit respiratoire, etc...) qui soit précise.

Au moins un mode de réalisation de l'invention a également pour objectif de fournir une telle technique qui soit aisément configurable par un utilisateur.

Au moins un mode de réalisation de l'invention a également pour objectif de fournir une telle technique qui fonctionne en temps réel.

Un objectif complémentaire d'au moins un mode de réalisation de l'invention est de fournir une telle technique qui soit peu coûteuse à mettre en œuvre.

Un objectif complémentaire d'au moins un mode de réalisation de l'invention est de fournir une telle technique qui ne nécessite pas de mettre en œuvre de techniques d'apprentissage automatique.

Un objectif complémentaire d'au moins un mode de réalisation de l'invention est de fournir une telle technique qui ne nécessite pas d'utiliser des bases de données.

Un objectif complémentaire d'au moins un mode de réalisation de l'invention est de fournir une telle technique qui peut être mise en œuvre sans employer de méthodes intrusives.

Un objectif complémentaire d'au moins un mode de réalisation de l'invention est de fournir une telle technique qui ne nécessite pas d'utiliser l'envoie d'un autre signal, une telle technique pouvant alors être qualifiée de technique passive.

Un objectif complémentaire d'au moins un mode de réalisation de l'invention est de fournir une telle technique qui soit simple à mettre en oeuvre.

Un objectif complémentaire d'au moins un mode de réalisation de l'invention est de fournir une telle technique qui ne nécessite pas d'utiliser une pluralité de capteurs.

Un autre objectif complémentaire d'au moins un mode de réalisation de l'invention est de fournir une telle technique qui puisse être appliquée à de nombreux types de signaux.

## 4. EXPOSÉ DE L'INVENTION

L'invention est défini par les revendications 1-11 annexées.

Ainsi, le principe général de l'invention consiste à réaliser un test d'hypothèse afin de détecter une telle anomalie.

Un tel procédé permet d'atteindre les objectifs précités. Ainsi, l'utilisation d'un tel procédé permet de détecter en temps réel des anomalies ce qui est crucial dans des applications médicales.

## 5. LISTE DES FIGURES

D'autres caractéristiques et avantages de l'invention apparaîtront à la lecture de la description suivante, donnée à titre d'exemple indicatif et non limitatif, et des dessins annexés, dans lesquels :

- la figure 1 présente une architecture simplifiée d'un dispositif de détection d'auto-PEEP selon un mode de réalisation particulier de l'invention ;
- la figure 2(a) présente des phases caractéristiques d'un signal au cours d'une respiration comprenant un inspiration et une expiration ;
- la figure 2(b) présente un signal représentatif de l'évolution du débit respiratoire d'un patient en fonction du temps, ledit signal comprenant une pluralité de cycles de respirations, ainsi que des instants d'intérêt pour la détection d'auto-PEEP ;
- la figure 3(a) présente des étapes mises en œuvre par un module de détection d'au moins un instant d'intérêt $t_k$ selon un mode de réalisation particulier de l'invention ;
- la figure 3(b) présente des étapes mises en œuvre par un module de détection d'au moins un instant d'intérêt $t_k$ selon un autre mode de réalisation particulier de l'invention ;
- les figures 4(a) et (b) présentent des courbes issues du traitement décrit en lien avec la figure 3(a);
- les figures 5(a) et (b) présentent des courbes issues du traitement décrit en lien avec la figure 3(b);
- la figure 6 présente des étapes mises en œuvre par un module d'estimation de paramètres selon un mode de réalisation particulier de l'invention ;
- les figures 7(a), (b) et (c) présentent, sous forme d'organigrammes, l'agencement des étapes mises en œuvre par un module de détection selon différents modes de réalisation de l'invention.

## 6. DESCRIPTION DÉTAILLÉE

Sur toutes les figures du présent document, les éléments et étapes identiques sont désignés par une même référence numérique.

Selon un mode de réalisation, l'invention est mise en œuvre au moyen de composants logiciels et/ou matériels. Dans cette optique, le terme "module" peut correspondre dans ce document aussi bien à un composant logiciel, qu'à un composant matériel ou à un ensemble de composants matériels et logiciels.

Un composant logiciel correspond à un ou plusieurs programmes d'ordinateur, un ou plusieurs sous-programmes d'un programme, ou de manière plus générale à tout élément d'un programme ou d'un logiciel apte à mettre en œuvre une fonction ou un ensemble de fonctions, selon ce qui est décrit ci-dessous pour le module concerné. Un tel composant logiciel est exécuté par un processeur de données d'une entité physique (terminal, serveur, passerelle, etc) et est susceptible d'accéder aux ressources matérielles de cette entité physique (mémoires, supports d'enregistrement, bus de communication, cartes électroniques d'entrées/sorties, interfaces utilisateur, etc).

De la même manière, un composant matériel correspond à tout élément d'un ensemble matériel (ou hardware) apte à mettre en œuvre une fonction ou un ensemble de fonctions, selon ce qui est décrit ci-dessous pour le module concerné. Il peut s'agir d'un composant matériel programmable ou avec processeur intégré pour l'exécution de logiciel, par exemple un circuit intégré, une carte à puce, une carte à mémoire, une carte électronique pour l'exécution d'un micrologiciel (firmware), etc.

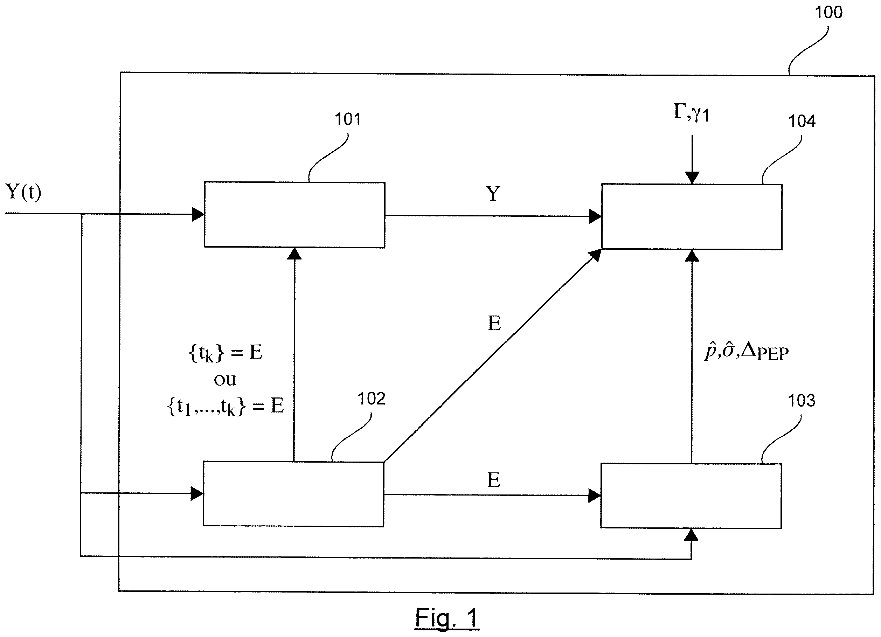

La figure 1 présente une architecture simplifiée d'un dispositif de détection d'auto-PEEP selon un mode de réalisation particulier de l'invention.

Un tel dispositif 100 de détection d'auto-PEEP comprend :

- un module 101 d'acquisition de données obtenues via la discrétisation, à partir d'une période d'échantillonnage $T_s$, d'un signal observé $Y(t) = \theta(t) + X(t)$ où le signal $X(t)$ est un bruit provenant des erreurs engendrées par des appareils de mesures, ou d'évènements parasites extérieurs, et où le signal $\theta(t)$est un signal d'intérêt. Plus précisément, il convient de noter que le signal d'intérêt $\theta(t) = f(t) + \Delta(t)$ où $f(t)$ est un signal de référence (i.e le signal sans anomalies, tel qu'il peut être observé chez un patient sain) et où $\Delta(t)$ correspond au signal représentatif des anomalies (i.e $\Delta(t)$peut être interprété comme étant un processus aléatoire qui représente la manifestation des anomalies qui surviennent chez un patient). Un tel module 101 permet aussi d'effectuer le formatage des données ainsi acquises (et plus précisément la sélection d'une sous partie des données reçues) en fonction d'un ensemble temporel $E$ (notamment un tel ensemble peut comprendre un ou plusieurs instants d'intérêt précis ou un tel ensemble $E$ peut être une plage temporelle d'intérêt i.e un intervalle temporel d'intérêt) fournit audit module 101 par un module 102 décrit ci-dessous;
- un module 102 de détermination d'un ensemble temporel $E$. Dans un mode de réalisation, le module 102 détermine au moins un instant d'intérêt précis $t_k$ à partir de données obtenues précédemment, ou des mêmes données acquises par le module 101 (ainsi on obtient un ensemble correspondant soit à $E = \{t_k\}$ ou $E = \{t_1, ..., t_K\}$ où $t_1, t_2, ..., t_K$ sont des instants particuliers non nécessairement consécutifs). Dans un mode de réalisation particulier, le module 102 fournit une plage temporelle d'intérêt $E = [t_0; t_0 + T]$ au module 101, où $t_0$ est un instant sélectionné par le module 102, et $T$ la longueur temporelle de la plage temporelle d'intérêt (ainsi, une telle plage temporelle correspond à un ensemble d'instants consécutifs) dans laquelle au moins un instant d'intérêt $t_k$ est compris. Le module 102 effectue un traitement particulier afin de détecter au moins un instant d'intérêt (i.e un ou plusieurs instants) en fonction de caractéristiques inhérentes au signal d'intérêt (par exemple, une telle caractéristique peut être liée à la présence d'un changement brusque comme une forte variation du signal d'intérêt lorsque celui-ci ne présente pas d'anomalies (i.e le signal de référence)). Ainsi, un instant d'intérêt appartenant à un ensemble $E = \{t_k\}$ ou $E = \{t_1, ..., t_K\}$ peut être l'instant à partir duquel un tel phénomène de variation se produit. La plage temporelle d'intérêt $E$ peut correspondre à la plage temporelle débutant à l'instant à partir duquel un tel phénomène de variation se produit et ayant une longueur donnée $T$ (i.e par exemple $t_0 = t_k$). Dans un mode de réalisation particulier de l'invention, le signal de référence $f(t)$ présente des motifs qui se répètent dans le temps (dans ce mode de réalisation, le module 102 n'a pas à connaître en détail le comportement du signal de référence $f(t)$ (i.e le module 102 ne possède pas de modèle du signal de référence $f(t)$). Dans un autre mode de réalisation, le module 102 possède un modèle du signal de référence $f(t)$;
- un module 103 d'estimation qui, à partir des mêmes données d'entrées que le module 101 (à savoir le signal échantillonné, ainsi que l'ensemble temporel $E$ issu du module 102 de détermination), permet d'estimer différents paramètres tels que la forme de la courbe du signal d'intérêt sur une plage temporelle d'intérêt (par exemple, la forme de la courbe du signal d'intérêt relative aux motifs qui se répètent comme mentionné ci-dessus), une déviation ou écart type, ou la valeur à un instant précis d'une plage temporelle ;
- un module 104 de détection d'une anomalie dans le signal d'intérêt $\theta(t)$(par exemple, une anomalie comme un Auto-PEEP, i.e lorsque $\Delta(t) \gg 0$). Le module 104 nécessite pour réaliser une telle détection des données formatées provenant du module 101, les estimations de différents paramètres issues du module 103 ainsi que des paramètres de configurations (qui sont un taux de fausses alarmes $\gamma_1$ tolérées et d'une valeur de tolérance $\tau$ dont l'utilité sera détaillée ultérieurement dans la présente demande). Ainsi, le module 104 met en oeuvre une technique de détection d'une anomalie au sein d'un signal d'intérêt $\theta(t)$par rapport à un signal de référence $f(t)$ en un ou plusieurs instants critiques précis $t_k$, ou sur une plage temporelle, et ce à partir de données provenant d'un signal observé $Y(t)$.

Il convient de noter que dans une variante de réalisation, les fonctionnalités des modules référencés 101, 102, 103 et 104, peuvent également être implémentés sous forme de matériel dans un composant programmable de type FPGA (« *Field Programmable Gate Array* » en anglais) ou de type ASIC (« *Application-Specific Integrated Circuit* » en anglais).

La figure 2(a) présente des phases caractéristiques d'un signal au cours d'une respiration comprenant un inspiration et une expiration.

Ainsi, au cours d'une respiration, on peut segmenter le signal correspond à l'évolution du débit d'air inspiré et expiré par le patient en trois plages temporelles distinctes en fonction du comportement d'un tel signal. Une telle segmentation correspond aux trois étapes d'une respiration, à savoir, une inspiration est réalisée au cours d'une première plage temporelle, puis, une pause se produit au cours d'une deuxième plage temporelle et une expiration est réalisée au cours d'une troisième plage temporelle. Ainsi, en lien avec la description du module 102, il convient de noter qu'une plage temporelle d'intérêt $E = [t_0; t_0 + T]$ peut être l'une de ces plages.

La figure 2(b) présente un signal représentatif de l'évolution du débit respiratoire d'un patient en fonction du temps, ledit signal comprenant une pluralité de cycles de respirations.

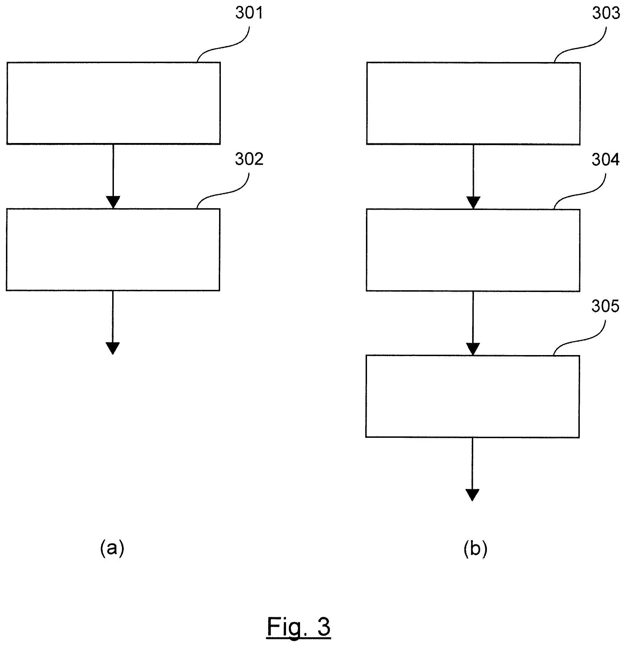

La figure 3(a) présente des étapes mises en œuvre par un module de détection d'au moins un instant d'intérêt $t_k$ selon un mode de réalisation particulier de l'invention.

Le module 102 met en œuvre, dans un mode de réalisation, une étape de décomposition du signal observé reçu via l'exécution d'une étape 301 d'application d'une transformée en ondelette sur le signal observé ($Y(t)$) puis l'exécution d'une étape de détection 302 d'une variation de changement des coefficients obtenus et du ou des instants correspondants, en détectant notamment le franchissement d'un seuil résultant de la mise en œuvre d'un test d'hypothèse. Ainsi, le module 102 peut détecter un ou plusieurs instants pour lesquels le comportement de la fonction de référence ou du signal d'intérêt a une caractéristique particulière (forte variation, etc...), et dès lors permet de définir une plage temporelle d'intérêt comprenant par exemple ce ou ces instants d'intérêt.

Plus précisément, sur une période temporelle donnée, généralement assez large, $[T_1;T_2]$ où $T_1$ et $T_2$ représente des temps, dans laquelle, on discrétise un signal observé avec une période d'échantillonnage $T_s$. Ainsi, on dispose, dans un mode de réalisation, d'un ensemble de points $y_n = Y(nT_s) = \theta(nT_s) + X(nT_s) = \delta_n + x_n$ avec $n$ un entier. Par exemple, on peut obtenir un échantillon de points d'une taille $L$ prédéterminée. Il convient de noter que la transformée discrète en ondelettes, appliquée lors de l'étape 301, permet de transformer $L$ éléments donnés définis dans le temps en $L$ coefficients.

Ainsi, en choisissant une base orthonormales d'ondelettes $g_i$, et du fait que la décomposition en ondelettes soit additive, la transformée discrète en ondelettes (mise en en oeuvre par le module 102) sur l'échantillon de taille $L$ du signal $Y = [y,...., y_L]$ permet d'obtenir $L$ coefficients (et permet de définir un vecteur $d = [d_1, ...., d_L]$) qui vérifient chacun l'équation suivante : $d_i = \alpha_i + \beta_i$ pour $i \in [\![1; L]\!]$ où $\alpha_i$ correspond à un coefficient d'ondelette d'intérêt et $\beta_i$ correspond à un bruit gaussien.

Ainsi, la matrice orthogonale $W$ associée à la transformée discrète en ondelettes soit permettre de vérifier les égalités suivantes : $d = WY$, $\alpha = W\delta$ et $\beta = WX$ où $\alpha = [\alpha_1,....,\alpha_L]$, $\beta = [\beta_1,....,\beta_L]$, $X = [x_1,....,x_L]$, $Y = [y_1,....,y_L]$, $\delta = [\delta_1,....,\delta_L]$ et la matrice $W$ a pour dimension $L \times L$. En fonction des hypothèses relatives au traitement des problèmes de frontières (« *edge problems* »), une telle matrice peut être orthogonale ou « presque orthogonale ». En considérant que le bruit $X(t)$ pour lequel on possède un échantillon $X = [x_1,....,x_L]$ est assimilable à un bruit gaussien (quand bien même le bruit $X(t)$ ne possède pas exactement les mêmes propriétés qu'un bruit gaussien), et puisque la base sur laquelle on projette est orthonormale, le bruit $X$ et le bruit $\beta$ ont les mêmes propriétés probabilistes. En effet, comme $\beta = WX$, $\beta$ hérite de la nature gaussienne de $X$, et $Cov(\beta,\beta) = E\beta^T\beta = EX^TW^TWX = Cov(X,X) = \sigma_x^2/$ où $\sigma_x$ est la déviation standard du bruit $X$.

En sortie de l'étape 301, on obtient donc un vecteur $d = [d_1,...., d_L]$ comprenant $L$ coefficients.

Il convient de noter que lorsque l'échantillon est grand (i.e $L$ est grand), la valeur absolue du bruit i.e la valeur absolue de l'un quelconque des $\beta_i$ est bornée, avec une forte probabilité, par une valeur seuil:

$$\lambda_u(L) == \sigma_X \sqrt{2 \ln L} = \sigma_\beta \sqrt{2 \ln L}$$ (dans un mode de réalisation particulier de l'invention, on peut choisir une autre valeur de seuil qui est proche comme, par exemple, $\lambda_u(L) = \sigma_X \sqrt{(2 \ln L - \ln(\ln L))}$ ou $\lambda_u(L) = 4\sigma_X$ (pour des échantillons grand ($L = 4000$))). Ce seuil peut aussi être interprétée comme étant la valeur minimale (en valeur absolue) du signal d'intérêt. Par conséquent, le problème de détection de pics, et donc d'instants d'intérêt associés, revient à effectuer le test d'hypothèse suivant : tester l'hypothèse ($H_0$) : $|d_i| > |\lambda_u(L)|$ par rapport à l'hypothèse ($H_1$) : $|d_i| \leq |\lambda_u(L)|$.

Ainsi, l'étape 302 de détection d'instants d'intérêt (et par conséquent éventuellement une plage temporelle d'intérêt) comprend les étapes suivantes :

- déterminer un premier seuil $\lambda_{SNT} = \sigma_X \lambda_{\gamma_2} (\frac{\lambda_u(L)}{\sigma_X})$ où $\lambda_{\gamma_2}$ ($\rho$) est l'unique solution en $\eta$ de l'équation suivante : $1 - [\Phi(\eta - \rho) - \Phi(-\eta - \rho)] = \gamma_2$, où la fonction $\Phi(.)$ est la fonction de répartition d'une variable aléatoire normale standard, et $\gamma_2$ est un taux de fausses alarmes tolérées ou acceptées. Ce taux est en général choisi comme étant très petit (par exemple $\gamma_2 = 10^{-4}$ ou $10^{-5}$ ou $10^{-7}$ ,...)

- comparer la valeur de $|d_i|$ avec la valeur du premier seuil $\lambda_{SNT}$. Lorsque la condition suivante est vérifiée : $|d_i| > \lambda_{SNT}$, à un instant donné, alors une déviation significative est détectée à cet instant. A contrario, lorsque la condition suivante est vérifiée : $|d_i| \leq \lambda_{SNT}$ alors aucune déviation significative n'est présente à cet instant donné.

Ainsi, en fonction de la configuration du module 102 par son utilisateur, il est possible de ne déterminer que quelques instants précis regroupés dans un ensemble temporel $E$. Il est aussi possible de définir une plage temporelle d'intérêt $E$ comprenant un tel instant détecté. En cas de détection de nombreux pics successifs, seul le premier instant sera considéré et les autres ne seront pas intégré à l'ensemble temporel.

Dans un autre mode de réalisation, le module 102 effectue un traitement préalable sur le signal observé ($Y(t)$) afin d'obtenir un signal observé lissé $\overline{Y(t)}$. Les étapes 301 et 302 sont appliquées sur un tel signal observé lissé $\overline{Y(t)}$.

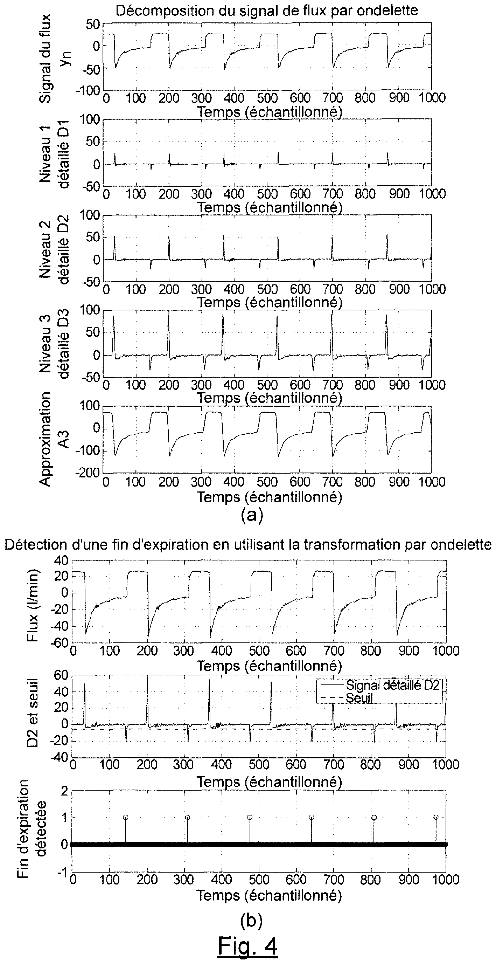

Les figures 4(a) et (b) présentent des courbes issues du traitement décrit en lien avec la figure 3(a).

La figure 3(b) présente des étapes mises en œuvre par un module de détection d'au moins un instant d'intérêt $t_k$ selon un autre mode de réalisation particulier de l'invention.

Le module 102 met en œuvre, selon ce mode de réalisation, une étape de filtrage appliquée au signal observé reçu. Ainsi, une telle étape permet de détecter un ou plusieurs instants pour lesquels le comportement de la fonction de référence ou du signal d'intérêt a une caractéristique particulière (forte variation, etc...). Ainsi, on peut aussi définir une plage temporelle d'intérêt via l'utilisation d'une telle étape de filtrage.

Plus précisément, et en lien avec un traitement d'un signal tel que présenté en figure 2(b), les instants d'intérêts correspondant aux instants relatifs à une fin d'expiration sont détectés automatiquement par le dispositif 100 via le module 102 mettant en œuvre une telle étape de filtrage.

Dans un mode de réalisation particulier de l'invention, une telle étape de filtrage comprend :

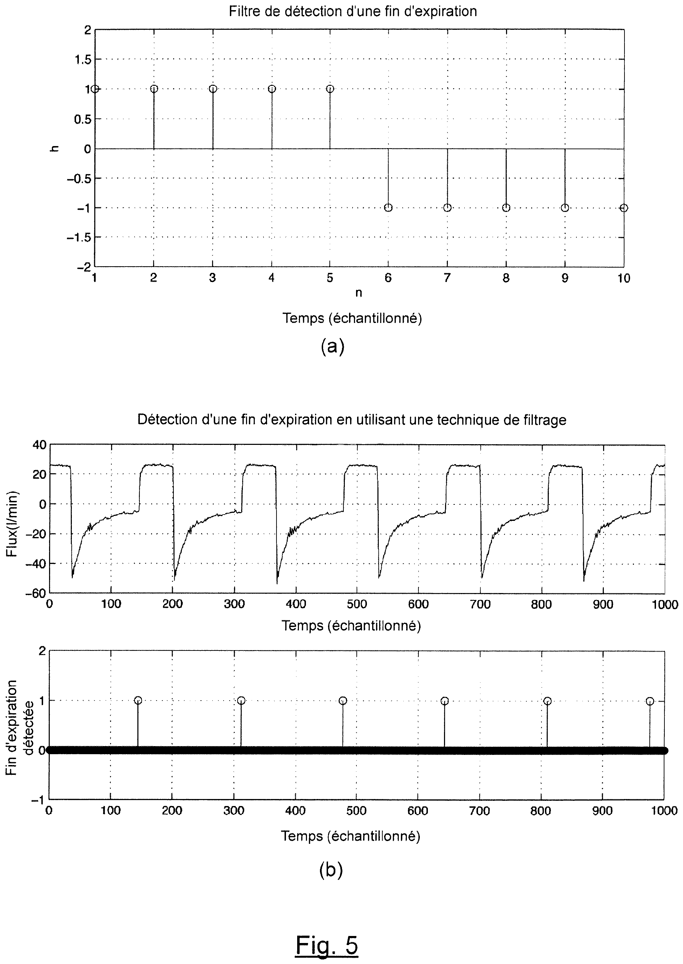

- une étape 303 de lissage du signal obtenu $Y(t) = \theta(t) + X(t)$. Ainsi, on obtient un signal observé lissé $\overline{Y(t)}$ ;
- une étape de détermination du signe 304 (positif ou négatif) du signal observé lissé $\overline{Y(t)}$ (dans le cas où le signal correspond à un débit ou un flux, on utilise le terme signe « positif » lorsque le flux d'air se produit selon une première direction, et signe « négatif » lorsque le flux d'air se produit selon une direction opposée à ladite première direction), en fonction d'une valeur de tolérance $\Delta$ permettant de corriger la valeur du signe obtenue (i.e si à un instant $t$, le signal observé lissé peut être considéré de prime abord comme « positif », mais que la valeur du signal observé lissé en cet instant est inférieure à une valeur de tolérance $\Delta$, alors le signal observé sera considéré comme «négatif».). Plus précisément, une telle étape consiste à déterminer l'évolution de la fonction $sign(\overline{Y(t)} - \Delta)$ où la fonction $sign(.)$ est la fonction déterminant un signe (positif ou négatif traduit respectivement par les valeurs +1 ou -1) . Il convient de noter que la valeur de tolérance $\Delta$ peut être défini au préalable par un clinicien, ou estimé à partir de représentation de la fonction de distribution du signal de flux ;
- une étape de détermination 305 des instants d'intérêts via l'application d'un filtre F' sur la fonction $sign(\overline{Y(t)} - \Delta)$ déterminée à l'étape précédente. Le filtre $F'$ correspond, par exemple, au filtre défini par $F' = [-1,....., -1,+1,......, +1]$ tel que présenté en figure 5(a). Un tel filtre permet de mettre en exergue la corrélation d'une pluralité d'échantillons. Ainsi, la figure 5(b), et plus précisément le graphique du bas de la figure 5(b), présente le résultat de la détermination de $F'^* sign(\overline{Y(t)} - \Delta)$ où les pics obtenus 501, 502, 503, 504, 505 et 506 correspondent aux instants à considérer.

Dans un autre mode de réalisation, lorsque le signal de référence $f(t)$ est périodique, il est possible d'obtenir une plage temporelle d'intérêt en effectuant une segmentation temporelle vis-à-vis du signal de référence $f(t)$ en utilisant des techniques basées sur les chaînes de Markov (par exemple la technique SSMM (pour « *Segmental Semi Markov Model* » en anglais) ou l'utilisation des chaînes de Markov cachées (HMM pour « *Hidden Markov Model* » en anglais)) qui sont mises en œuvre dans le module 102.

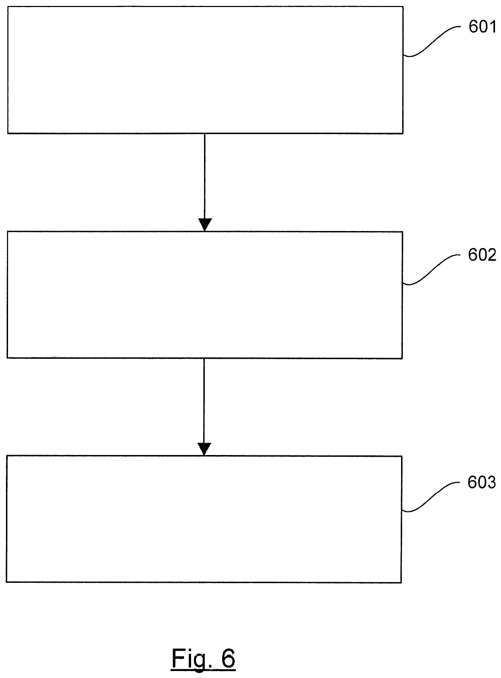

La figure 6 présente des étapes mises en œuvre par un module d'estimation de paramètres selon un mode de réalisation particulier de l'invention.

Le module 103 à partir de données relatives au signal observé ainsi que d'un ensemble temporel provenant du module 102 exécute plusieurs étapes d'estimations de paramètres qui doivent être ensuite transmis au module de décision 104.

Le module 103 permet, dans un mode de réalisation particulier de l'invention, de réaliser :

- une estimation 601 d'un vecteur $p$ représentatif de la forme du signal de référence $f(t)$ sur un intervalle de temps donné en lien avec l'ensemble temporel $E$;
- une estimation 602 de la déviation standard $\sigma_x$ du bruit $X(t)$ ;
- une estimation 603 d'une référence $\Delta_{PEP}$ permettant de définir, à partir d'une valeur de tolérance donné $\tau_0$, une valeur de tolérance corrigée $\tau_0 + \Delta_{PEP}$.

  L'étape d'estimation 601 du vecteur $p$ représentatif de la forme du signal de référence $f(t)$ sur un intervalle de temps donné comprend:

- une étape d'obtention du modèle du signal de référence $f(t)$ sur un intervalle de temps donné comprenant des paramètres inconnus (par exemple, sur l'intervalle de temps correspondant à la fin de la phase d'expiration d'air par un patient, un tel signal de référence est modélisé par la fonction $f(x) = a - be^{-ct}$ où $b>0$ et $c>0$. Les paramètres inconnus sont les paramètres $a$, $b$ et $c$) ;
- une étape d'application d'une technique de régression (non linéaire) sur les données observées sur l'intervalle de

7

temps considéré. Ainsi, à partir d'un ensemble de points d'observation $(t_i, y_i)$, on détermine la valeur des paramètres indéterminés (en l'occurrence, concernant la fonction $f(x) = a - be^{-ct}$, on détermine les paramètres $(a,b,c)$ solution

$$\arg\min_{a,b,c} \sum_{i=1}^{L} w_i (y_i - (a - be^{-ct_i}))^2$$

du problème d'optimisation suivant , où les poids $w_i$ sont choisis de sorte qu'il réduise l'influence de certains points.

- Une étape de détermination du vecteur $p$ à partir du résultat de l'étape précédente.

Dans un deuxième mode de réalisation, lorsque le signal de référence $f(t)$ présente des motifs répétitifs dans le temps, l'étape d'estimation 601 du vecteur $p$ représentatif de la forme du signal de référence $f(t)$ sur un intervalle de temps donné comprend une étape de détermination d'une estimation d'un tel vecteur $\hat{p}$ à partir d'un échantillon de données sur un intervalle de temps sur lequel aucune anomalie n'est présente. Par exemple, à partir d'un échantillon de $2L+1$ éléments (pour un seul cycle de respiration), on a le vecteur $\hat{p} = u_1 = [\hat{p}_{-L}, ...., \hat{p}_0, .., \hat{p}_L]^T$ qui correspond à

$$\left[ \frac{\hat{\theta}(t_k - LT_s)}{\hat{\theta}(t_k)}, ...., 1, .., \frac{\hat{\theta}(t_k + LT_s)}{\hat{\theta}(t_k)} \right]^T$$

et $\hat{\theta}(t_k \pm kT_s) = \hat{f}(t_k \pm kT))$. Pour obtenir une estimation plus precise, on effectue cette étape de détermination pour $K$ cycles de respiration (sans anomalies) et l'estimation $\hat{p}$ correspond à la moyenne des estimations obtenues.

Ainsi, on a
$$\hat{p} = \frac{1}{K} \sum_{i=1}^{K} u_i$$

Dans un troisième mode de réalisation, l'estimation 601 du vecteur $p$ peut être faite de manière dynamique.

Plus précisément, dans un mode de réalisation de l'invention, le vecteur est modifié en prenant en compte un

$$\hat{p} = \frac{1-\mu}{1-\mu^K} \sum_{i=1}^{K} \mu^{K-i} u_i$$

paramètre $\mu \in [0;1]$ permettant de limiter l'importance d'estimation "ancienne". Ainsi, on obtient .

L'estimation 602 de la déviation standard $\sigma_X$ du bruit $X(t)$ peut être réalisée selon l'une quelconque des deux étapes décrites ci-dessous.

La première étape consiste à effectuer une estimation via l'application d'une technique de régression, et à considérer les résidus obtenus comme du bruit.

Plus précisément, pour un seul cycle de respiration, à partir d'un échantillon de $2L+1$ éléments, l'échantillon

$$\hat{\sigma}_X = \sigma_1 = \frac{1}{2L} \sqrt{\sum_{i=0}^{2L} (f(t_k - (L-i)T_s) - \hat{\theta}(t_k - (L-i)T_s))^2}$$

étant on obtient une valeur .

Pour obtenir une estimation plus précise, de la même manière que pour l'estimation du vecteur de forme, il convient d'effectuer la moyenne des valeurs de la déviation obtenues pour K cycles de respiration (sans anomalies).

$$\hat{\sigma}_X = \frac{1}{K} \sum_{i=1}^{K} \sigma_i$$
Ainsi,

Dans un troisième mode de réalisation, l'estimation 602 de la déviation standard $\sigma_X$ du bruit $X(t)$ peut être faite de manière dynamique.

Plus précisément, dans un mode de réalisation de l'invention, le vecteur est modifié en prenant en compte un paramètre $\mu \in [0;1]$ permettant de limiter l'importance d'estimation "ancienne". Ainsi, on obtient

$$\hat{\sigma}_X = \frac{1-\mu}{1-\mu^K} \sum_{i=1}^{K} \mu^{K-i} \sigma_i$$
.

La deuxième étape consiste à effectuer une estimation à partir des coefficients d'ondelette en utilisant soit un estimateur du type MAD (« *Median Absolute Deviation* »), soit un estimateur du type DATE (« *d-dimensional adaptative trimming estimator* »), lorsque le bruit $X(t)$ est un bruit blanc gaussien, ou peut être considéré comme assimilable à un bruit blanc gaussien. Ces deux estimateurs (MAD ou DATE) qui utilisent des coefficients d'ondelette ne nécessitent pas l'obtention du modèle de la fonction $f$ contrairement à la technique précédente.

L'étape d'estimation 603 d'une référence $\Delta_{PEP}$ permet de définir, à partir d'une valeur de tolérance donné $\tau_0$, une valeur de tolérance corrigée $\tau = \tau_0 + \Delta_{PEP}$ qui sera utilisée par le module 104.

Plus précisément, la référence $\Delta_{PEP}$ peut être obtenue en observant, en un point d'intérêt $t_k$, les valeurs du signal d'intérêt sur plusieurs cycles de respiration sans anomalies, et en choisissant $\Delta_{PEP}$ comme étant la valeur moyenne de celles-ci. De plus, cette valeur de tolérance corrigé doit être validée par le clinicien. Elle est le reflet d'un certain degré de limitation de débit lié aux réglages du ventilateur (réglage d'une pression expiratoire positive - PEP - extrinsèque).

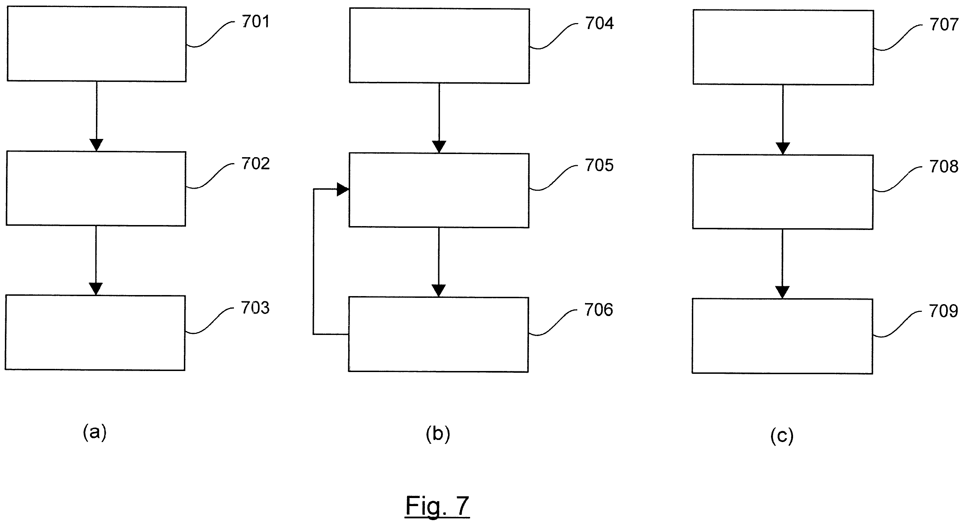

Les figures 7(a), (b) et (c) présentent, sous forme d'organigrammes, l'agencement des étapes mises en œuvre par un module de détection de détection selon différents modes de réalisation de l'invention.

La figure 7(a) présente, sous forme d'un organigramme, l'agencement des étapes mises en œuvre par un module de détection, selon un mode de réalisation de l'invention, pour détecter à un instant précis $t_k$, une anomalie dans le signal d'intérêt.

Le problème, relatif à la détection d'une déviation entre le signal d'intérêt $\theta(t)$ et le signal de référence $f(t)$ à un instant critique choisi $t_k$, ladite déviation étant considérée comme telle en fonction d'une valeur de tolérance $\tau$ peut être formulé sous la résolution d'un test permettant de trancher entre deux hypothèses, $H_0$ et $H_1$, dont une et une seule est vraie, au vu du signal observé $Y(t)$ formaté obtenu via le module 101. La valeur de tolérance $\tau$ est donc une valeur pour laquelle on considère qu'une déviation est ou non réalisée. Ainsi, on considère que lorsque la différence (ou l'écart), en valeur absolue, entre le signal d'intérêt $\theta(t)$ et le signal de référence $f(t)$ à un instant critique choisi $t_k$ est supérieure à la valeur de tolérance $\tau$ alors une déviation s'est produite. A contrario, on considère que lorsque la différence (ou l'écart), en valeur absolue, entre le signal d'intérêt $\theta(t)$ et le signal de référence $f(t)$ à un instant critique choisi $t_k$ est inférieure ou égale à la valeur de tolérance $\tau$ alors une déviation (ou anomalie) ne s'est pas produite. Ainsi, la valeur de tolérance $\tau$ permet de ne pas considérer de petites variations, marginales et sans importance, du signal d'intérêt $\theta(t)$ vis-à-vis du signal de référence $f(t)$ à un instant critique choisi $t_k$. Le choix de la valeur de tolérance est fonction à la fois de la valeur de données antérieures, ainsi, que des connaissances du praticien (cf. la description de l'étape 603).

Ainsi, il convient, lors d'un de la réalisation d'un tel test, de choisir entre les deux hypothèses suivantes au vu du signal observé $Y(t)$ formaté : l'hypothèse $H_0$ est que l'on a $|\theta(t_k) - f(t_k)| > \tau$ et l'hypothèse $H_1$ est que l'on a $|\theta(t_k) - f(t_k)| \le \tau$.

Dans un mode de réalisation de l'invention, l'instant critique choisi $t_k$ étant connu (via par exemple l'utilisation du module 102), le module 101 peut établir un formatage de $2L+1$ échantillons du signal observé au voisinage de l'instant critique choisi $t_k$ et fournit un tel échantillon de données au module 104. Dans un mode de réalisation, les échantillons ne sont pas répartis de manière uniforme autour de l'instant critique choisi $t_k$. Dans un mode de réalisation préférentiel, il est choisi de centrer les $2L+1$ échantillons de part et d'autres de l'instant critique choisi $t_k$. Ainsi, en supposant que l'on choisisse une période d'échantillonnage $T_s$, le module 104 obtient dans un mode de réalisation préférentiel, du module 101, les $2L+1$ échantillons du signal observé $Y(t)$, mis sous la forme d'un vecteur colonne $Y = [Y(t_k - LT_k),....,Y(t_k - T_s), Y(t_k), Y(t_k + T_s),.., Y(t_k + LT_s)]^T$. De par la définition du signal observé, il vient l'équation vectorielle suivante $Y = \Theta + \Omega$ où $\Theta = [\theta(t_k - LT_s),....,\theta(t_k),..,\theta(t_k + LT_s)]^T$ et $\Omega = [X(t_k - LT_s),...., X(t_k),.., X(t_k + LT_s)]^T$.

Lorsque l'on peut établir que $\Theta = [\theta(t_k - LT_s),....,\theta(t_k),..,\theta(t_k + LT_s)]^T = p.\theta(t_k)$ avec

$$p = \left[ p_{-L},....,p_0,..,p_L \right]^T = \left[ \frac{\theta(t_k - LT_s)}{\theta(t_k)},....,1,..,\frac{\theta(t_k + LT_s)}{\theta(t_k)} \right]^T$$ et où le vecteur $p$ est connu (car obtenu soit via l'estimation réalisée par le module 103), on réalise une étape de projection de sorte que l'on ait :

$$\frac{p^T Y}{\|p\|_2^2} = \frac{p^T(\Theta + X)}{\|p\|_2^2} = \frac{p^T(p\theta(t_k) + X)}{\|p\|_2^2} = \theta(t_c) + \frac{p^T X}{\|p\|_2^2}$$ où la fonction $\|.\|_2$ est la norme euclidienne standard.

En posant $u = \dfrac{p^T Y}{\|p\|_2^2}$ et $w = \dfrac{p^T X}{\|p\|_2^2}$, l'équation est simplifiée de la manière suivante : $u = \theta(t_k) + w$. L'étape 701 consiste à déterminer la valeur de $u$ en utilisant notamment l'estimation du vecteur $p$ fournie par le module 103.

Ainsi, grâce à l'utilisation du vecteur $p$ (pour réaliser la projection), ou plus précisément de son estimation, le problème au départ multidimensionnel devient un problème en dimension 1.

En remarquant que le problème de test d'hypothèse reste le même que précédemment, à savoir tester l'hypothèse $H_0 : |\theta(t_k) - f(t_k)| > \tau$ contre l'hypothèse $H_1 : |\theta(t_k) - f(t_k)| \le \tau$, et en utilisant des données « projetées » (i.e $u$) et en remarquant que la variance du bruit $w = \dfrac{p^T X}{\|p\|_2^2}$ est plus petite que celle du bruit en $t_k$, le test consiste alors à effectuer une comparaison de la valeur de $|u|$ avec un seuil de discrimination $\lambda_{\gamma_1}^*$( qui est fonction du taux de fausses alarmes $\gamma_1$ tolérées ou acceptées par le praticien, et d'une valeur de tolérance $\tau$ ) qui est obtenu dans une étape 702 décrite ci-dessous. Ainsi, lorsque la condition suivante est vérifiée : $|u| > \lambda_{\gamma_1}^*$ alors une déviation significative est détectée à l'instant $t_k$ au sein du signal d'intérêt, en respectant le taux de fausses alarmes. A contrario, lorsque la condition suivante est vérifiée : $|u| \leq \lambda_{\gamma_1}^*$ alors aucune déviation significative n'est présente à l'instant $t_k$ au sein du signal d'intérêt, en respectant le taux de fausses alarmes. Une telle comparaison est réalisée au cours de l'étape 703.

L'étape de détermination 702 du seuil de discrimination $\lambda_{\gamma_1}^*$ consiste à évaluer la fonction quantile $Q$ de la variable aléatoire $|\tau + w|$ en la valeur $(1-\gamma_1)$. Pour rappel, la fonction quantile $Q$ de la variable aléatoire $|\tau + w|$ est définie de la manière suivante : $Q_{|\tau+w|}(u) = \inf(\{x \,/\, F_{|\tau+w|}(x) \geq u\})$ où la fonction $F_{|\tau+w|}$ correspond à la fonction de répartition de la variable aléatoire $|\tau + w|$, i.e la fonction $F_{|\tau+w|}$ est définie de la manière suivante : $F_{|\tau+w|}(x) = P(|\tau + w|) \leq x)$.

Dès lors, l'obtention du seuil de discrimination est réalisée via le calcul suivant : $\lambda_{\gamma_1}^* = Q_{|\tau+w|}(1-\gamma_1)$.

Dans un mode de réalisation, lorsque $w$ est un bruit gaussien centré, le seuil de discrimination est déterminé de la manière suivante : $\lambda_{\gamma_1}^* = \sigma_w \lambda_{\gamma_1}\left(\dfrac{\tau}{\sigma_w}\right)$ où la fonction $\lambda_{\gamma_1}(\rho)$ est définie comme étant l'unique solution $\eta$ de l'équation suivante: $1-[\Phi(\eta - \rho)-\Phi(-\eta - \rho)] = \gamma_1$, où la fonction $\Phi(.)$ est la fonction de répartition d'une variable aléatoire normale standard.

La figure 7(b) présente, sous forme d'un organigramme, l'agencement des étapes mises en œuvre par un module de détection, selon un mode de réalisation de l'invention, pour détecter en une pluralité d'instant précis $t_k$, une anomalie.

Lorsque le module 104 souhaite détecter la présence d'une anomalie en une pluralité d'instants précis $t_k$ avec $k \in [\![1; K]\!]$ compris dans l'ensemble temporel $E$, il convient de vérifier si les anomalies se réalisant à ces instants sont ou non corrélées. Lorsque celles-ci ne sont pas corrélées, il suffit d'appliquer de manière itérative les étapes décrites en lien avec la figure 7(a).

En revanche, lorsque celles-ci sont corrélées (i.e lorsque, à ces instants, des motifs répétitifs similaires sont présents) , il est possible d'utiliser cette information pour améliorer le procédé de détection, au sens où l'on réduit la probabilité de détection de fausses alarmes, et que l'on augmente la probabilité de détection d'anomalies.

Dans ce mode de réalisation, on considère que les valeurs de référence en chacun des instants $t_k$ est identique (à savoir $f = f(t_1) = f(t_2) = ... = f(t_K)$) (il est toujours possible de se ramener à un tel mode de réalisation, quand bien même les valeurs des $f(t_i)$ ne sont pas identiques. En effet, il suffit de choisir une valeur $\overline{f}$ comme étant la moyenne des valeur $f(t_i)$ et à considérer que $\overline{f} = f(t_1) = f(t_2)= ... = f(t_K)$) . En utilisant la même technique de projection que celle décrite en lien avec la figure 7(a), on obtient $u_k = \theta(t_k) + w_k$ pour $k \in [\![1; K]\!]$ puis en moyennant on obtient $u_{1:K} = \theta_{1:K} + w_{1:K}$ avec

$$u_{1:K} = \frac{1}{K}\sum_{k=1}^{K} u_k \,,\quad \theta_{1:K} = \frac{1}{K}\sum_{k=1}^{K} \theta(t_k) \quad \text{et} \quad w_{1:K} = \frac{1}{K}\sum_{k=1}^{K} w_k \,.$$

En supposant que le signal de référence ne varie pas trop en les K instants $t_k$ avec $k \in [\![1; K]\!]$, la détection d'une anomalie peut être vue comme un test d'hypothèse entre les deux hypothèses suivantes :

$$(\mathrm{H}_0) : \left|\theta_{1:K} - f\right| > \tau$$

$$(\mathrm{H}_1) : \left|\theta_{1:K} - f\right| \leq \tau$$

En fonction d'un taux de fausses alarmes $\gamma$ tolérées ou acceptées par le praticien, la règle de décision est définie de la manière suivante :

Si $|u_{1:K}| > \lambda_{1:K}^{(h)}$ alors une anomalie est détectée ;
Si $|u_{1:K}| \leq \lambda_{1:K}^{(l)}$ alors aucune déviation n'est détectée ;
Si $\lambda_{1:K}^{(l)} < |u_{1:K}| \leq \lambda_{1:K}^{(h)}$ alors aucune décision ne peut être prise en l'espèce. La prise de décision est reportée jusqu'à un instant suivant.
Le seuil supérieur $\lambda_{1:K}^{(h)}$ est dérivé de la condition $1 - F_{|\tau+w_{1:K}|}(\lambda_{1:K}^{(h)}) = \gamma_1$.
Le seuil inférieur $\lambda_{1:K}^{(l)}$ est dérivé de la condition $1 - F_{|\tau+w_{1:K}|}(\lambda_{1:K}^{(l)}) = 1 -\gamma_1$.
Où la fonction $F_{|\tau-w_{1:K}|}(.)$ correspond à la fonction de répartition de la variable aléatoire $\|\tau + w_{1:K}\|$. Ainsi, les deux seuils sont calculés de la manière suivante :

$$\lambda_{1:K}^{(h)} = Q_{|\tau + w_{1:K}|}(1 - \gamma_1)$$

$$\lambda_{1:K}^{(l)} = Q_{|\tau + w_{1:K}|}(\gamma_1)$$

où $Q_{|\tau + w_{1:K}|}(.)$ est la fonction quantile correspondante.

Lorsque la variable $w_{1:K}$ est centrée et gaussienne, on obtient les formules explicites ci-dessous :

$$\lambda_{1:K}^{(h)} = \sigma_{w_{1:K}} \lambda_{\gamma_1}\left(\frac{\tau}{\sigma_{w_{1:K}}}\right)$$

$$\lambda_{1:K}^{(l)} = \sigma_{w_{1:K}} \lambda_{1-\gamma_1}\left(\frac{\tau}{\sigma_{w_{1:K}}}\right)$$

où $\lambda_\gamma(\rho)$ est l'unique solution en de l'équation :

$$1 - \left[\Phi(\eta - \rho) - \Phi(-\eta - \rho)\right] = \gamma_1$$

et $\lambda_{1-\gamma_1}(\rho)$ est l'unique solution en de l'équation : $1 - [\Phi(\eta - \rho) - \Phi(-\eta - \rho)] = 1 - \gamma_1$, et la fonction $\Phi(.)$ est la fonction de répartition d'une variable aléatoire normale standard. On considère que l'élément $\sigma_{w_{1:K}}$ est obtenu via le module d'estimation 103.

Ainsi, le procédé de détection d'une anomalie comprend :

- une étape 704 de détermination d'initialisation de variables : $j := 1$ ;
- une étape 705 de détermination des éléments suivantes : $|u_{1:j}|$, $\lambda_{1:j}^{(h)}$ et $\lambda_{1:j}^{(l)}$ ;
- une étape de comparaison 706 consistant à réaliser les opérations suivantes en un instant $t_j$.

Si $|u_{1:j}| > \lambda_{1:j}^{(h)}$ alors une anomalie est détectée ;
Si $|u_{1:j}| \leq \lambda_{1:j}^{(l)}$ alors aucune déviation n'est détectée ;
Si $\lambda_{1:j}^{(l)} < |u_{1:j}| \leq \lambda_{1:j}^{(h)}$ alors aucune décision ne peut être prise en l'espèce. La prise de décision est reportée au test réalisé à un instant suivant. Ainsi, dans ce cas de figure, on incrémente la variable $j$ (i.e $j := j+1$), et on réitère les étapes 705 et 706 jusqu'au traitement de $|u_{1:K}|$ si aucune des comparaisons précédentes n'a aboutie à la détection d'une anomalie.

Pour qu'un tel procédé de décision ne soit pas trop long à être exécuté, il est préférable de limiter le nombre d'itération pour qu'une décision soit prise jusqu'à un nombre $M$, et de procéder en dernier lieu à un test final consistant à comparer $|u_{1:M}|$ avec uniquement $\lambda_{1:M}^{(h)}$.

Si $|u_{1:M}| > \lambda_{1:M}^{(h)}$ alors, une anomalie est détectée, si $|u_{1:M}| \leq \lambda_{1:M}^{(h)}$ alors aucune anomalie est détectée.

La figure 7(c) présente, sous forme d'un organigramme, l'agencement des étapes mises en œuvre par un module de détection, selon un mode de réalisation de l'invention, pour détecter sur un intervalle temporel ou une plage temporelle $E = [t_0 ; t_0 + T]$ une anomalie.

Dans ce mode de réalisation, on considère que le module de décision 104 obtient :

- un échantillon de $L$ données du signal observé, $Y = [y_1, ...., y_L]$, en provenance du module 101 ;
- un échantillon de $L$ données du signal de référence, $F = [f_1, ...., f_L]$ (où $f_k = f(k.T_s)$), obtenu soit via le module d'estimation 103 (si le signal de référence est périodique), soit si on possède une modélisation du signal de référence, via l'application d'un tel modèle. Ainsi, ce mode de réalisation nécessite l'utilisation d'un échantillon du modèle de référence contrairement aux deux autres modes de réalisation.

Le problème, relatif à la détection d'une déviation entre le signal d'intérêt $\theta(t)$ et signal de référence $f(t)$ sur un intervalle temporel donné $E = [t_0 ; t_0 + T]$, revient à réaliser le test d'hypothèse suivant consistant à choisir entre les deux hypothèses suivantes au vu du signal observé $Y(t)$ formaté : l'hypothèse $H_0$ est que l'on a $\|Y - F\| > \tau$ et l'hypothèse $H_1$ est que l'on a $\|Y - F\| \leq \tau$.

On choisit une norme de Malahanobis définie pour un vecteur v, de dimension L, de la manière suivante : $\|v\|$ = $(v^T C^{-1} v)^{1/2}$ où C est la matrice de covariance du bruit du signal.

Dans un mode de réalisation de l'invention, on considère que cette matrice est connue.

Dans un autre mode de réalisation de l'invention, on considère que cette matrice est obtenue via une étape d'estimation, en supposant que le bruit du signal est coloré.

En fonction du taux de fausses alarmes $\gamma$ tolérées ou acceptées par le praticien, on peut détecter une anomalie sur l'intervalle temporel donné $E = [t_0; t_0 + T]$ en comparant, lors d'une étape 709, la valeur de $\|Y - F\|$, obtenue lors d'une étape 707, avec un seuil $\lambda_{\gamma_1}^*$ qui est déterminé dans une étape 708 décrite ci-dessous. Ainsi, lorsque $\|Y - F\| > \lambda_{\gamma_1}^*$, cela signifie qu'une anomalie est présente sur l'intervalle temporel donné $E = [t_0; t_0 + T]$. Et, a contrario, lorsque $\|Y - F\| \leq \lambda_{\gamma_1}^*$, cela signifie qu'aucune anomalie est présente sur l'intervalle temporel donné $E = [t_0; t_0 + T]$.

Le seuil $\lambda_{\gamma_1}^*$ déterminé lors de l'étape 708 est dérivé de la condition suivante : $1 - F_{\|\Delta + X\|}(\lambda_\gamma^*) \leq \gamma_1$ pour tout $\Delta$ vérifiant $\|\Delta\| \leq \tau$, où la fonction $F_{\|\Delta + X\|}$ correspond à la fonction de répartition de la variable aléatoire $\|\Delta + X\|$.

Ainsi, l'étape 708 de détermination du seuil $\lambda_{\gamma_1}^*$ comprend une étape de détermination de l'unique élément $\eta$ de l'équation $1 - R(\rho, \eta) = \gamma_1$, correspondant à $\lambda \gamma_1(\rho)$, puis une étape de détermination de $\lambda_{\gamma_1}^* = \lambda_{\gamma_1}(\tau)$ et où la fonction $R(\rho, .)$ correspond à la fonction de répartition de la racine carrée d'une variable aléatoire quelconque suivant une loi de distribution du $\chi^2$ non centrée avec $L$ degrés de liberté et définie par le paramètre $\rho^2$.

## Revendications

1. Procédé de détection de la présence d'une anomalie $\Delta(t)$ comprise dans un signal physique représentatif d'une évolution de paramètres physiologiques d'un patient, dit signal physique observé $Y(t)$, ledit signal observé $Y(t)$ comprenant une addition d'un signal physique de perturbation $X(t)$ et d'un signal de référence $f(t)$, et ladite anomalie étant relative à une modification du comportement du signal de référence $f(t)$ par rapport à une première valeur de tolérance $\tau$, ledit procédé étant mis en œuvre par un dispositif (100) de détection de la présence d'une anomalie et comprenant :

   - une étape de détermination (102) d'un ensemble temporel $E$ comprenant au moins un instant d'intérêt **caractérisé en ce que** le procédé comprend:

      - une étape de détection de la présence de ladite anomalie comprise dans ledit signal physique observé $Y(t)$ comprenant :
      - une étape de projectionselon un vecteur de forme $p$ du signal de référence, du signal physique observé, ladite étape de projection délivrant une valeur projetée $u$ pour ledit au moins un instant d'intérêt, le vecteur de forme $p$ du signal de référence étant estimé sur l'ensemble temporel $E$ au moyen d'une technique de régression à partir d'un modèle du signal de référence, et
      - une étape de comparaison, pour au moins un instant d'intérêt . d'une valeur absolue de ladite valeur projetée $u$ avec un premier seuil $\lambda_{\gamma_1}^* = Q|\tau + w|(1 - \gamma_1)$ où $\gamma_1$ est ledit taux de fausses alarmes tolérées, $\tau$ est ladite première valeur de tolérance, $Q$ est une fonction quantile d'une variable aléatoire $|\tau + w|$ où w est une projection dudit signal physique de perturbation selon l'estimation dudit vecteur de forme $p$, ladite étape de comparaison correspondant à un test d'hypothèse permettant de détecter une anomalie lorsque la valeur absolue de ladite valeur projetée $u$ est supérieure audit premier seuil $\lambda_{\gamma_1}^*$

2. Procédé de détection selon la revendication 1 **caractérisé en ce que** l'étape de détermination (102) comprend :

   - une étape d'application (301) d'une transformée en ondelette sur le signal observé pendant un temps d'observation, ladite étape d'application (301) délivrant des coefficients ;
   - une étape de comparaison (302) des valeurs absolues desdits coefficients avec un deuxième seuil $\lambda_{SNT} = \sigma_x \lambda_\gamma$ (a) où une fonction $\lambda_\gamma(\rho)$ est l'unique solution en $\eta$ d'une équation $1 - [\Phi(\eta - \rho) - \Phi(-\eta - \rho)] = \gamma_2$, où une fonction $\Phi(.)$ est la fonction de répartition d'une variable aléatoire normale standard, $\gamma$, est un deuxième taux de fausses alarmes tolérées, $\sigma_x$ est la déviation du bruit $X(t)$, $L$ une taille de l'échantillon dudit signal observé, et $a$ une valeur proche de $\sqrt{2 \ln L}$, ladite étape de comparaison délivrant au moins un instant d'intérêt lorsque la valeur absolue d'un desdits coefficients est supérieure audit deuxième seuil $\lambda_{SNT}$.

3. Procédé de détection selon la revendication 1 **caractérisé en ce que** l'étape de détermination (102) comprend une étape de filtrage.

**4.** Procédé de détection selon l'une quelconque des revendications 1 à 3 **caractérisé en ce qu'**il comprend en outre une étape de lissage du signal observé.

**5.** Procédé de détection selon l'une quelconque des revendications 1 à 4, **caractérisé en ce que** lorsque ledit ensemble $E$ comprend $K$ instants d'intérêt et que des valeurs du signal physique source sont corrélées en ces instants, ladite étape de détection comprend :

- une étape de projection, selon un vecteur de forme $p$ du signal de référence, du signal physique observé, ladite étape de projection délivrant une valeur projetée $u_i$ pour chaque instant d'intérêt;
- une première étape d'initialisation d'une variable $j$ à un ;
- une deuxième étape d'initialisation d'une variable $u_{1:j}$ correspond à une moyenne de $j$ valeurs projetées ;
- une étape de détermination (705) des éléments suivantes : $|u_{1:j}|$ , $\lambda_{1:j}^{(h)}$ et $\lambda_{1:j}^{(l)}$ avec $\lambda_{1:j}^{(h)} = Q_{|\tau+w_{1:j}|}(1-\gamma_1)$ , $\lambda_{1:j}^{(l)} = Q_{|\tau+w_{1:j}|}(\gamma_1)$ où $Q_{|\tau+w_{1:j}|}(.)$ est la fonction quantile de la variable aléatoire $|\tau+w_{1:j}|$ où $w_{1:j}$ correspond à une moyenne de $j$ projections dudit signal physique de perturbation selon ledit vecteur de forme $p$;
- une étape de comparaison (706) comprenant des comparaisons entre lesdits éléments déterminés, pour un instant $t_j$ , et lorsque $|u_{1:j}|> \lambda_{1:j}^{(h)}$ alors une anomalie est détectée, lorsque $|u_{1:j}|\leq\lambda_{1:j}^{(l)}$ alors aucune déviation n'est détectée, et lorsque $\lambda_{1:j}^{(l)}<|u_{1:j}|\leq\lambda_{1:j}^{(h)}$ la variable $j$ est incrémentée et la deuxième étape d'initialisation et les étapes de détermination et de comparaison sont réitérées.

**6.** Procédé de détection selon la revendication 5, **caractérisé en ce que** le nombre d'itérations de la deuxième étape d'initialisation et des étapes de détermination et de comparaison est limité par une valeur donnée $M$, inférieure à $K,$ et **en ce qu'**un test final consistant à comparer $|u_{1:M}|$ avec uniquement $\lambda_{1:M}^{(h)}$ est réalisé, le test détectant une anomalie lorsque $|u_{1:M}| > \lambda_{1:M}^{(h)}$.

**7.** Procédé de détection selon l'une quelconque des revendications 1 à 4, **caractérisé en ce que** lorsque ledit ensemble $E$ correspond à un intervalle temporel, ladite étape de détection comprend :

- une étape d'obtention d'un échantillon, d'une taille $L,$ du signal observé ;
- une étape d'obtention d'un échantillon, d'une taille $L,$ du signal de référence ;
- une étape de détermination d'une valeur correspondant à une norme de la différence des deux échantillons obtenus ;
- une étape de comparaison de ladite valeur avec un troisième seuil $\lambda_{\gamma_1}^* = \lambda_{\gamma_1}(\tau)$ où la fonction $\lambda_{\gamma_1}(\rho)$ correspond à l'unique solution, en $\eta$, de l'équation $1 - R(\rho,\eta) = \gamma_1$, où la fonction $R(\rho,.)$ correspond à la fonction de répartition de la racine carrée d'une variable aléatoire quelconque suivant une loi de distribution du $\chi^2$ non centrée avec $L$ degrés de liberté et définie par le paramètre $\rho^2$ , ladite étape de comparaison correspondant audit test d'hypothèse et permettant de détecter une anomalie lorsque ladite valeur est supérieure audit troisième seuil $\lambda_{\gamma_1}^* = \lambda_{\gamma_1}(\tau)$.

**8.** Procédé de détection selon l'une quelconque des revendications 1 à 7, **caractérisé en ce que** ledit signal observé correspond à un signal appartenant au groupe comprenant : un signal dit électrocardiogramme, un signal dit électroencéphalogramme, un signal représentatif d'une évolution d'une pression artérielle, un signal représentatif d'une évolution d'une concentration en oxygène dans des tissus, un signal représentatif d'une évolution d'une pression intracrânienne, un signal représentatif de l'évolution d'un débit respiratoire.

**9.** Procédé de détection selon l'une quelconque des revendications 1 à 8, **caractérisé en ce que** ledit signal physique de perturbation $X(t)$ est gaussien,

**10.** Produit programme d'ordinateur, comprenant des instructions de code de programme pour la mise en œuvre du procédé selon au moins une des revendications 1 à 9, lorsque ledit programme est exécuté sur un ordinateur.

**11.** Dispositif de détection de la présence d'une anomalie $\Delta(t)$ comprise dans un signal représentatif d'une évolution de paramètres physiologiques d'un patient, dit signal physique physique observé $Y(t)$, ledit signal observé comprenant une addition d'un signal physique de perturbation $X(t)$, et d'un signal de référence $f(t)$ , et ladite anomalie étant relative à une modification du comportement du signal de référence $f(t)$ par rapport à une première valeur de tolérance $\tau$, ledit dispositif comprenant:

- des moyens de détermination (102) d'un ensemble temporel $E$ comprenant au moins un instant d'intérêt **caractérisé en ce que** le dispositif comprend:

- des moyens de détection de la présence de ladite anomalie comprise dans ledit signal physique observé *Y(t)* comprenant :
- des moyens de projectionselon un vecteur de forme *p* du signal de référence, du signal physique observé, ladite étape de projection délivrant une valeur projetée *u* pour ledit au moins un instant d'intérêt, le vecteur de forme *p* du signal de référence étant estimé sur l'ensemble temporel *E* au moyen d'une technique de régression à partir d'un modèle du signal de référence par un module d'estimation (103), et
- des moyens de comparaison, pour au moins un instant d'intérêt, d'une valeur absolue de ladite valeur projetée *u* avec un premier seuil $\lambda_{\gamma1}^* = Q_{|\tau+w|}(1 - \gamma_1)$ où $\gamma_1$ est ledit taux de fausses alarmes tolérées, $\tau$ est ladite première valeur de tolérance, *Q* est une fonction quantile d'une variable aléatoire $|\tau + w|$ où *w* est une projection dudit signal physique de perturbation selon l'estimation dudit vecteur de forme *p*, ladite comparaison correspondant à un test d'hypothèse permettant de détecter une anomalie lorsque la valeur absolue de ladite valeur projetée *u* est supérieure audit premier seuil $\lambda_{\gamma1}^*$

## Patentansprüche

1. Verfahren zur Erfassung des Vorliegens mindestens einer Anomalie $\Delta(t)$, die in einem physischen Signal enthalten ist, das für eine Entwicklung von physiologischen Parametern eines Patienten, beobachtetes physisches Signal *Y(t)* genannt, repräsentativ ist, wobei das beobachtete Signal *Y(t)* eine Addition eines physischen Störsignals *X(t)* und eines Referenzsignals *f(t)* umfasst, und wobei sich die Anomalie auf eine Änderung des Verhaltens des Referenzsignals *f(t)* in Bezug auf einen ersten Toleranzwert $\tau$ bezieht, wobei das Verfahren von einer Vorrichtung (100) zur Erfassung des Vorliegens einer Anomalie eingesetzt wird und umfasst:

   - einen Schritt der Bestimmung (102) einer Zeiteinheit *E,* umfassend mindestens einen Zeitpunkt von Interesse;
   **dadurch gekennzeichnet, dass** das Verfahren umfasst:

      - einen Schritt der Erfassung des Vorliegens der Anomalie, die in dem beobachteten physischen Signal *Y(t)* enthalten ist, umfassend:
      - einen Schritt der Projektion des beobachteten Signals gemäß einem Vektor der Form *p* des Referenzsignals, wobei der Projektionsschritt einen projizierten Wert *u* für den mindestens einen Zeitpunkt von Interesse liefert, wobei der Vektor der Form *p* des Referenzsignals über die Zeiteinheit *E* mit Hilfe einer Regressionstechnik auf Basis eines Modells des Referenzsignals geschätzt wird, und
      - einen Schritt des Vergleichs, für mindestens einen Zeitpunkt von Interesse, eines Absolutwerts des projizierten Werts *u* mit einer ersten Grenze $\lambda_{y1}^* = Q_{|\tau+w|}(1-y_1)$, wobei $y_1$ die Rate von tolerierten Fehlalarmen ist, $\tau$ der erste Toleranzwert ist, *Q* eine Quantilfunktion einer Zufallsvariablen $|\tau+w|$ ist, wobei *w* eine Projektion des physischen Störsignals gemäß der Schätzung des Vektors der Form *p* ist, wobei der Vergleichsschritt einem Hypothesetest entspricht, der es ermöglicht, eine Anomalie zu erfassen, wenn der Absolutwert des projizierten Vektors *u* größer als die erste Grenze $\lambda_{\gamma1}^*$ ist.

2. Erfassungsverfahren nach Anspruch 1, **dadurch gekennzeichnet, dass** der Bestimmungsschritt (102) umfasst:

   - einen Schritt des Anwendens (301) einer Wavelet-Transformation an dem beobachteten Signal für eine Beobachtungszeit, wobei der Anwendungsschritt (301) Koeffizienten liefert;
   - einen Schritt des Vergleichens (302) der Absolutwerte der Koeffizienten mit einer zweiten Grenze $\lambda_{SNT}=\sigma_x \cdot \lambda_y,(a)$, wobei eine Funktion $\lambda_\gamma,(p)$ die einzige Lösung bei $\eta$ einer Gleichung $1-[\Phi(\eta-p)-\Phi(-\eta-p)]=y_2$ ist, wobei eine Funktion $\Phi(.)$ die Verteilungsfunktion einer normalen Standard-Zufallsvariablen ist, y eine zweite Rate von tolerierten Fehlalarmen ist, $\sigma_x$ die Ablenkung des Lärms *X(t)* ist, *L* eine Größe der Probe des beobachteten Signals ist, und *a* ein Wert nahe $\sqrt{2\ln L}$ ist, wobei der Vergleichsschritt mindestens einen Zeitpunkt von Interesse liefert, wenn der Absolutwert eines der Koeffizienten größer als die zweite Grenze $\lambda_{SNT}$ ist.

3. Erfassungsverfahren nach Anspruch 1, **dadurch gekennzeichnet, dass** der Bestimmungsschritt (102) einen Filterschritt umfasst.

4. Erfassungsverfahren nach einem der Ansprüche 1 bis 3, **dadurch gekennzeichnet, dass** es ferner einen Glättungsschritt des beobachteten Signals umfasst.

5. Erfassungsverfahren nach einem der Ansprüche 1 bis 4, **dadurch gekennzeichnet, dass**, wenn die Einheit *E K* Zeitpunkte von Interesse umfasst, und Werte des physischen Quellensignals zu diesen Zeitpunkten korreliert sind, der Erfassungsschritt umfasst:

- einen Schritt der Projektion des beobachteten physischen Signals gemäß einem Vektor der Form $p$ des Referenzsignals, wobei der Projektionsschritt einen projizierten Wert $u_1$ für jeden Zeitpunkt von Interesse liefert;
- einen ersten Schritt der Initialisierung einer Variablen $j$ auf Eins;
- einen zweiten Schritt der Initialisierung einer Variablen $u_{1:j}$ entsprechend einem Durchschnitt von $j$ projizierten Werten;
- einen Schritt der Bestimmung (705) der folgenden Elemente: $|u_{1:j}|$, $\lambda_{1:j}^{(h)}$ und $\lambda_{1:j}^{(1)}$, wobei $\lambda_{1:j}^{(h)}=Q_{|\tau+w1:j|}(1-y_1)$, $\lambda_{1:j}^{(1)}=Q_{|\tau+w1:j|}(y_1)$, wobei $Q_{|\tau+w1:j|}(.)$ die Quantilfunktion der Zufallsvariablen $|\tau+w_{1:j}|$ ist, wobei $w_{1:j}$ einem Durchschnitt von $j$ Projektionen des physischen Störsignals gemäß dem Vektor der Form $p$ entspricht;
- einen Vergleichsschritt (706), umfassend Vergleiche zwischen den bestimmten Elementen für einen Zeitpunkt $t_j$, und wenn $|u_{1:j}|>\lambda_{1:j}^{(h)}$, wird eine Anomalie festgestellt, wenn $|u_{1:j}|\leq\lambda_{1:j}^{(1)}$, wird keine Abweichung festgestellt, und wenn $\lambda_{1:j}^{(1)}< |u_{1:j}|\leq\lambda_{1:j}^{(h)}$, wird die Variable j inkrementiert, und der zweite Initialisierungsschritt und die Bestimmungs- und Vergleichsschritte werden reiteriert.

6. Erfassungsverfahren nach Anspruch 5, **dadurch gekennzeichnet, dass** die Anzahl von Iterationen des zweiten Initialisierungsschritts und der Bestimmungs- und Vergleichsschritte durch einen gegebenen Wert $M$, kleiner als $K$, begrenzt ist, und dass ein Endtest durchgeführt wird, der darin besteht, $|u_{1:M}|$ nur mit $\lambda_{1:M}^{(h)}$ zu vergleichen, wobei der Test eine Anomalie feststellt, wenn $|u_{1:M}|>\lambda_{1:M}^{(h)}$.

7. Erfassungsverfahren nach einem der Ansprüche 1 bis 4, **dadurch gekennzeichnet, dass**, wenn die Einheit $E$ einem Zeitintervall entspricht, der Erfassungsschritt umfasst:

- einen Schritt des Erhalts einer Probe der Größe $L$ des beobachteten Signals,
- einen Schritt des Erhalts einer Probe der Größe $L$ des Referenzsignals,
- einen Schritt der Bestimmung eines Werts entsprechend einer Norm der Differenz der zwei erhaltenen Proben;
- einen Schritt des Vergleichs des Werts mit einer dritten Grenze $\lambda_{y1}^* = \lambda_{y1}(\tau)$, wobei die Funktion $\lambda_{y1}(p)$ der einzigen Lösung bei $\eta$ der Gleichung $1-R(p,\eta) = y_1$ entspricht, wobei die Funktion $R(p,.)$ der Verteilungsfunktion der Quadratwurzel einer beliebigen Zufallsvariablen gemäß einem Verteilungsgesetz des $X^2$, nicht zentriert mit $L$ Freiheitsgraden und durch den Parameter $p^2$ definiert, entspricht, wobei der Vergleichsschritt dem Hypothesetest entspricht und es ermöglicht, eine Anomalie festzustellen, wenn der Wert größer als die dritte Grenze $\lambda_{y1}^* = \lambda_{y1}(\tau)$ ist.

8. Erfassungsverfahren nach einem der Ansprüche 1 bis 7, **dadurch gekennzeichnet, dass** das beobachtete Signal einem Signal entspricht, der der Gruppe angehört, umfassend: ein so genanntes Elektrokardiogramm-Signal, ein so genanntes Elektroenzephalogramm-Signal, ein Signal, das für eine Entwicklung eines arteriellen Drucks repräsentativ ist, ein Signal, das für eine Entwicklung einer Sauerstoffkonzentration in Geweben repräsentativ ist, ein Signal, das für eine Entwicklung eines intrakranialen Drucks repräsentativ ist, ein Signal, das für die Entwicklung eines Atemflusses repräsentativ ist.

9. Erfassungsverfahren nach einem der Ansprüche 1 bis 8, **dadurch gekennzeichnet, dass** das physische Störsignal $X(t)$ ein Gauss'sches Signal ist.

10. Computerprogrammprodukt, umfassend Programmcodebefehle für den Einsatz des Verfahrens nach mindestens einem der Ansprüche 1 bis 9, wenn das Programm auf einem Computer ausgeführt wird.

11. Vorrichtung zur Erfassung des Vorliegens einer Anomalie $\Delta(t)$, die in einem physischen Signal enthalten ist, das für eine Entwicklung von physiologischen Parametern eines Patienten, beobachtetes physisches Signal $Y(t)$ genannt, repräsentativ ist, wobei das beobachtete Signal $Y(t)$ eine Addition eines physischen Störsignals $X(t)$ und eines Referenzsignals $f(t)$ umfasst, und wobei sich die Anomalie auf eine Änderung des Verhaltens des Referenzsignals $f(t)$ in Bezug auf einen ersten Toleranzwert $\tau$ bezieht, wobei die Vorrichtung umfasst:

- Mittel zur Bestimmung (102) einer Zeiteinheit $E$, umfassend mindestens einen Zeitpunkt von Interesse;
**dadurch gekennzeichnet, dass** die Vorrichtung umfasst:

- Mittel zur Erfassung des Vorliegens der Anomalie, die in dem beobachteten physischen Signal $Y(t)$ enthalten ist, umfassend:

- Mittel zur Projektion des beobachteten Signals gemäß einem Vektor der Form *p* des Referenzsignals, wobei der Projektionsschritt einen projizierten Wert *u* für den mindestens einen Zeitpunkt von Interesse liefert, wobei der Vektor der Form *p* des Referenzsignals über die Zeiteinheit *E* mit Hilfe einer Regressionstechnik auf Basis eines Modells des Referenzsignals durch ein Schätzmodul (103) geschätzt wird, und

- Vergleichsmittel, für mindestens einen Zeitpunkt von Interesse, eines Absolutwerts des projizierten Werts *u* mit einer ersten Grenze $\lambda_{y1}^* = Q_{|\tau+w|}(1-y_1)$, wobei $y_1$ die Rate von tolerierten Fehlalarmen ist, $\tau$ der erste Toleranzwert ist, Q eine Quantilfunktion einer Zufallsvariablen $|\tau+w|$ ist, wobei w eine Projektion des physischen Störsignals gemäß der Schätzung des Vektors der Form *p* ist, wobei der Vergleich einem Hypothesetest entspricht, der es ermöglicht, eine Anomalie zu erfassen, wenn der Absolutwert des projizierten Vektors *u* größer als die erste Grenze $\lambda_{y1}^*$ ist.

**Claims**

1. Method for detecting the presence of an anomaly $\Delta(t)$ included in a physical signal representing the progress of a patient's physiological parameters, called observed physical signal *Y(t)*, said observed physical signal *Y(t)* comprising an addition of a physical disturbance signal *X(t)*, and a reference signal *f(t)*, and said anomaly being relative to a modification of the behavior of the reference signal *f(t)* relative to a first tolerance value ($\tau$), said method being carried out by a device (100) capable of detecting an anomaly and comprising a step for determining (102) a temporal set *E* comprising at least one instant of interest;
   **characterized in that** said method comprises :
   a step for detecting the presence of said anomaly within said observed physical signal *Y(t)* comprinsing :

   - a step for projecting the observed physical signal along a vector of form *p* of the reference signal, said step for projecting delivering a projected value *u* for said at least one instant of interest, said vector of form *p* of the reference signal is obtained for the temporal set *E* by using a regression technique on the basis of a model of the reference signal; and
   - a step for comparing, for at least one instant of interest, an absolute value of said projected value *u* with a first threshold $\lambda_{\gamma_1}^* = Q_{|\tau+w|}(1 - \gamma_1)$ where $\gamma_1$ is a rate of tolerated false alarms, $\tau$ is said first tolerance value, *Q* is a quantile function of a random variable $|\tau + w|$ where w is a projection of said physical disturbance signal according to the estimation of said vector of form *p*, said step for comparing corresponding to a hypothesis test enabling the detection of an anomaly when the absolute value of said projected value *u* is above said first threshold $\lambda_{\gamma_1}^*$.

2. Method for detecting according to claim 1 **characterized in that** the step for determining (102) comprises:

   - a step for applying (301) a wavelet transform to the observed signal during a time of observation, said step for applying (301) delivering coefficients;
   - a step for comparing (302) absolute values of said coefficients with a second threshold $\lambda_{SNT} = \sigma_X \lambda_{y2}(a)$ where a function $\lambda_{y2}(\rho)$ is the unique solution in $\eta$ of an equation $1-[\Phi(\eta - \rho) - \Phi(-\eta - \rho)] = \gamma_2$, where a function $\Phi(.)$ is the distribution function of a standard normal random variable, $\gamma_2$ is a second rate of tolerated false alarms, $\sigma_X$ is the deviation of the noise *X(t)*, *L* is a size of the sample of said observed signal and *a* is a value close to $\sqrt{2 \ln L}$ , said step for comparing delivering at least one instant of interest when the absolute value of one of said coefficients is above said second threshold $\lambda_{SNT}$.

3. Method for detecting according to claim 1 **characterized in that** the step for determining (102) comprises a filtering step.

4. Method for detecting according to any one of the claims 1 to 3 **characterized in that** it further comprises a step for smoothing the observed signal.

5. Method for detecting according to any one of the claims 1 to 4, **characterized in that**, when said set *E* comprises *K* instants of interest and when the values of the source physical signal are correlated with these instants, said step for detecting comprises:

   - a step for projecting the observed physical signal along a vector of form *p* of the reference signal, said step for projecting delivering a projected value $u_i$ for each instant of interest;
- a first step for initializing a variable $j$ at one;
- a second step for initializing a variable $u_{1:j}$ corresponding to an average of $j$ projected values;
- a step for determining (705) the following elements: $|u_{1:j}|$, $\lambda_{1:j}^{(h)}$ and $\lambda_{1:j}^{(l)}$ with $\lambda_{1:j}^{(h)} = Q_{|\tau+w_{1:j}|}(1-\gamma_1)$, $\lambda_{1:j}^{(l)} = Q_{|\tau+w_{1:j}|}(\gamma_1)$ where $Q|_{\tau+w_{1:j}|}(.)$ is the quantile function of the random variable $|\tau+w_{1:j}|$ I where $w_{1:j}$ corresponds to an average of $j$ projections of said physical disturbance signal along said vector of form $p$;
- a step for comparing (706) comprising comparisons between said determined elements for an instant $t_j$, and when $|u_{1:j}| > \lambda_{1:j}^{(h)}$ then an anomaly is detected, when $|u_{1:j}| \leq \lambda_{1:j}^{(l)}$ then no deviation is detected and when $\lambda_{1:j}^{(l)} < |u_{1:j}| \leq \lambda_{1:j}^{(h)}$ then the variable $j$ is incremented and the second step for initializing and the steps for determining and comparing are reiterated.

6. Method for detecting according to claim 5, **characterized in that** the number of iterations of the second step for initializing and of the steps for determining and comparing is limited by a given value $M$, smaller than $K$, and **in that** a final test consisting in comparing $|u_{1:M}|$ with only $\lambda_{1:M}^{(h)}$ is done, the test detecting an anomaly when $|u_{1:M}| > \lambda_{1:M}^{(h)}$.

7. Method for detecting according to any one of the claims 1 to 4, **characterized in that** when said set $E$ corresponds to a time span, said step for detecting comprises:

   - a step for obtaining a sample, sized $L$, of the observed signal;
   - a step for obtaining a sample, sized $L$, of the reference signal, ;
   - a step for determining a value corresponding to a norm of the difference between the two samples obtained;
   - a step for comparing said value with a third threshold $\lambda_{\gamma_1}^* = \lambda_{\gamma_1}(\tau)$ where the function $\lambda_{\gamma_1}(\rho)$ corresponds to the unique solution, in $\eta$, of the equation $1 - R(\rho, \eta) = \gamma_1$, where the function $R(\rho,.)$ corresponds to the distribution function of the square root of any unspecified random variable according to a non-centered $\chi^2$ distribution law with $L$ degrees of freedom and defined by the parameter $\rho^2$, said step for comparing corresponding to said hypothesis test and enabling the detection of an anomaly when said value is greater than said third threshold $\lambda_{\gamma_1}^* = \lambda_{\gamma_1}(\tau)$.

8. Method for detecting according to any one of the claims 1 to 7, **characterized in that** said observed signal corresponds to a signal belonging to the group comprising: a signal called an electrocardiogram signal, a signal called a electroencephalogram signal, a signal representing a progress of arterial pressure, a signal representing a progress of a concentration of oxygen in the tissues, a signal representing a progress of intra-cranial pressure, a signal representing the progress of a respiratory flow.

9. Method for detecting according to any one of the claims 1 to 8, **characterized in that** said physical disturbance signal $X(t)$ is Gaussian.

10. Computer program product comprising program code instructions for the implementing of the method according to at least one of the claims 1 to 9, when said program is executed by a computer.

11. Device for detecting the presence of an anomaly $\Delta(t)$ included in a physical signal representing the progress of a patient's physiological parameters, called observed physical signal $Y(t)$, said observed physical signal $Y(t)$ comprising an addition of a physical disturbance signal $X(t)$, and a reference signal $f(t)$, and said anomaly being relative to a modification of the behavior of the reference signal $f(t)$ relative to a first tolerance value $(\tau)$, said device comprising means for determining (102) a temporal set $E$ comprising at least one instant of interest;
**characterized in that** said device comprises :
means for detecting the presence of said anomaly within said observed physical signal $Y(t)$ comprinsing :

   - means for projecting the observed physical signal along a vector of form $p$ of the reference signal, said step for projecting delivering a projected value $u$ for said at least one instant of interest, said vector of form $p$ of the reference signal is obtained for the temporal set $E$ by using a regression technique on the basis of a model of the reference signal; and
   - means for comparing, for at least one instant of interest, an absolute value of said projected value $u$ with a first threshold $\lambda_{\gamma_1}^* = Q_{|\tau+w|}(1-\gamma_1)$ where $\gamma_1$ is a rate of tolerated false alarms, $\tau$ is said first tolerance value, $Q$ is a quantile function of a random variable $|\tau + w|$ where w is a projection of said physical disturbance signal according to the estimation of said vector of form $p$, said step for comparing corresponding to a hypothesis test enabling the detection of an anomaly when the absolute value of said projected value $u$ is above said first threshold $\lambda_{\gamma_1}^*$.

Fig. 1

$Y(t)$

101

$Y$

$\Gamma, \gamma_1$

104

$\{t_k\} = E$
ou
$\{t_1,...,t_k\} = E$

102

$E$

$\hat{p}, \hat{\sigma}, \Delta_{PEP}$

103

$E$

100

EP 2 828 796 B1

18

Segmentation d'une phase de respiration

(a)

Signal du flux

(b)

## Fig. 2

301

302

(a)

303

304

305

(b)

Fig. 3

Décomposition du signal de flux par ondelette

(a)

Détection d'une fin d'expiration en utilisant la transformation par ondelette

(b)

Fig. 4

Filtre de détection d'une fin d'expiration

Temps (échantillonné)

(a)

Détection d'une fin d'expiration en utilisant une technique de filtrage

Temps (échantillonné)

Temps (échantillonné)

(b)

## Fig. 5

601

602

603

<u>Fig. 6</u>

707

708

709

(c)

704

705

706

(b)

Fig. 7

701

702

703

(a)

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2010147305 A
- EP 2281506 A1
- US 2005232096 A1, VAN HELVOIRT JAN

**Littérature non-brevet citée dans la description**

- **Y. ZHU.** Automatic Detection of Anomalies in Blood Glucose Using a Machine Learning Approach. *IEEE International Conference on Information Reuse and Integration (IRI),* 2010
- **WULSIN et al.** Semi-Supervised Anomaly Detection for EEG Waveforms Using Deep Belief Nets. *Ninth International Conférence on Machine Learning and Applications (ICMLA),* 2010
- **R.J. RIELLA et al.** Method for automatic detection of wheezing in lung sounds. *Brazilian Journal of Médical and Biological Research,* 2009, vol. 42, 674-684
- *Détection et caractérisation en temps différé de sauts de dynamique: application à la segmentation de l'EMG,* 01 Janvier 1995, 1189-1192